(19) Europäisches Patentamt

European Patent Office

Office européen des brevets

(11) **EP 0 955 033 B1**

(12) **EUROPÄISCHE PATENTSCHRIFT**

(45) Veröffentlichungstag und Bekanntmachung des Hinweises auf die Patenterteilung:
**03.03.2004 Patentblatt 2004/10**

(51) Int Cl.[7]: **A61K 7/06**

(21) Anmeldenummer: **99107147.3**

(22) Anmeldetag: **13.04.1999**

(54) **Haarbehandlungsmittel mit alpha-Hydroxycarbonsäureestern und Polymeren**

Hair treatment composition comprising alpha-hydroxy carboxylic acid esters and polymers

Composition pour le traitement des cheveux contenant des esters d'acides alpha-hydroxy carboxyliques et des polymères

(84) Benannte Vertragsstaaten:
**DE ES FR GB IT**

(30) Priorität: **06.05.1998 DE 19820109**

(43) Veröffentlichungstag der Anmeldung:
**10.11.1999 Patentblatt 1999/45**

(73) Patentinhaber: **Wella Aktiengesellschaft**
**64295 Darmstadt (DE)**

(72) Erfinder:
• **Pasquier, Gilbert**
**1724 Praroman (CH)**
• **Irrgang, Bernard, Dr.**
**1713 St. Anton (CH)**
• **Karlen, Thomas, Dr.**
**3013 Bern (CH)**
• **Chambettaz, Daniel**
**1717 St. Ursen (CH)**
• **Steinbrecht, Karin, Dr.**
**64372 Ober-Ramstadt (DE)**

(56) Entgegenhaltungen:
WO-A-99/04756          CH-A- 443 565
DE-A- 19 621 681          US-A- 4 719 930

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Gegenstand der Erfindung ist ein Haarbehandlungsmittel, insbesondere ein Mittel zum Stylen von Haaren, das bestimmte alpha-Hydroxycarbonsäureester in Kombination mit filmbildenden, haarfestigenden Polymeren enthält sowie die Verwendung der genannten Ester in Haarstylingmitteln.

Es sind bereits zahlreiche Produkte bekannt, welche den Haaren durch Polymerzusatz Halt, Volumen, Elastizität, Sprungkraft und Glanz verleihen. Diese Stylingprodukte erleichtern als Gel die Formgebung, verbessern als Haarspray den Stand und als Festigerschaum das Volumen des Haares. Darüber hinaus sollen Stylingprodukte dem Haar neben natürlicher Sprungkraft und Elastizität einen natürlichen Glanz verleihen.

Zur Erreichung dieser Ziele werden häufig Silikone als Additive zur Beeinflussung der Griffeigenschaften und des Glanzes eingesetzt. Der Nachteil dieser Substanzklasse liegt jedoch in ihrer Tendenz, die Haare zu belasten, insbesondere falls silikonhaltige Präparate mehrmals nacheinander angewendet werden. Dieses Problem ist als sogenannter "build-up"-Effekt bekannt. Dabei bildet sich ein Mantel von hypdrophobem Silikonmaterial um das Haar, der Schmutz anzieht und der auch durch Waschen der Haare oftmals nicht vollständig entfernt werden kann.

In der CH 443 565 wird die Verwendung von Milchsäureestern als Emulgiermittel in kosmetischen Produkten in Form von wässrigen Emulsionen beschrieben. Beschrieben wird auch ein Haarspray mit Gehalt an Lauryllactat und Polyvinylpyrrolidon. In der DE 196 21 681 A1 werden wässrige Perlglanzkonzentrate beschrieben mit einem Gehalt an Estern von mehrwertigen, hydroxysubstituierten Carbonsäuren mit Fettalkoholen mit 6-22 C-Atomen, z.B. Weinsäuremonocetearylester. Die Perlglanzkonzentrate können in Shampoos eingesetzt werden, welche zusätzlich Dimethylpolysiloxan enthalten. In der US 4,719,930 werden Haarbehandlungsmittel beschrieben, welche Cetyllactat und bestimmte monoquaternäre Ammoniumverbindungen enthalten, z.B. Tris(oligooxyethyl)octadecylammoniumphosphat. In der WO 99/04756 werden Hautpflege- und Körperreinigungsmittel beschrieben mit einem Gehalt an einer Mischung von kurzkettigen, C2- bis C10-alpha-Hydroxyestern und langkettigen, C11- bis C24-alpha-Hydroxyestern. Eine Zusammensetzung enthält z.B. Octadecyl-(S)-lactat, Octyl-(S)-lactat und einen Polyacrylatverdicker vom Typ Carbopol 980. In der WO 99/03447 werden haarkonditionierende Zusammensetzungen beschrieben mit einem Gehalt an Estern von alpha- oder beta-Hydroxysäuren, insbesondere eine Mischung aus Isostearylcitrat, Isostearylglycolat, Isostearyllactat und Isostearylmalat. Die Ausführungsbeispiele enthalten zusätzlich die Polymere Polyquaternium-37, Polysilicone-8 bzw. Hydroxyethylcellulose.

Es bestand somit die Aufgabe ein Haarbehandlungsmittel, insbesondere ein Haarstylingmittel zur Verfügung zu stellen, mit welchem sowohl ein gutes Haarstyling möglich ist und welches weitgehend die positiven Griff und Glanzeigenschaften von silikonhaltigen Mitteln aufweist, das sich jedoch durch einfaches Waschen der Haare mit einem Shampoo wieder entfernen läßt und das die Haare möglichst wenig belastet.

Gelöst wird die Aufgabe durch ein Haarbehandlungsmittel mit einem Gehalt an

(A) mindestens einem Ester, welcher mindestens eine alpha-Hydroxycarbonsäuregruppe enthält, die mit einem Alkohol verestert ist, der 8 bis 18 C-Atome aufweist und
(B) mindestens einem filmbildenden und haarfestigenden Polymer;

wobei der Ester (A) die allgemeine Formel (I) aufweist

$$HO\text{-}C(XR^1)(YR^2)\text{-}CO_2R^3 \tag{I}$$

wobei $R^1$ die Gruppe $CO_2R^4$ bedeutet, $R^2$ ausgewählt ist aus H und $CO_2R^4$, $R^3$ und $R^4$ gleich oder verschieden sein können und ausgewählt sind aus verzweigten oder unverzweigten Alkylgruppen mit 8 bis 18 C-Atomen, wobei die Alkylgruppen weitere Substituenten wie Hydroxygruppen, Aminogruppen oder Halogene tragen können und X und Y gleich oder verschieden sein können und entweder eine Einfachbindung darstellen oder ausgewählt sind aus verzweigten oder unverzweigten Alkylengruppen mit vorzugsweise 1 bis 5 C-Atomen,, wobei die Alkylengruppen weitere Substituenten wie Hydroxygruppen, Aminogruppen oder Halogene tragen können;
und/oder wobei das haarfestigende Polymer (B) nichtionisch, kationisch, anionisch oder amphoter sein kann, wobei nichtionische synthetische Polymere ausgewählt sind aus Homopolymeren des N-Vinylformamids, Copolymeren aus Vinylpyrrolidon und Vinylacetat, Terpolymeren aus Vinylpyrrolidon, Vinylacetat und Vinylpropionat, Polyacrylamiden, Polyvinylalkoholen und Polyethylenglykol/Polypropylenglykol Copolymeren;
ausgenommen einem Haarbehandlungsmittel mit einem Gehalt an 0,3 Gew.% einer Mischung aus Isostearylcitrat, Isostearylglycolat, Isostearyllactat und Isostearylmalat in Kombination mit entweder 2 Gew.% Polyquaternium-37, 0,5 Gew.% Polysilicone-8 oder 0,75 Gew.% Hydroxyethylcellulose.
Es handelt sich um ein gut auswaschbares Mittel zur Behandlung und Festigung von Haaren, welches in seinen Eigenschaften gegenüber den vorteilhaften anwendungstechnischen Eigenschaften (insbesondere Griff, Glanz, Belastung und Auswaschbarkeit) der bisher bekannten silikonhaltigen Produkte mindestens vergleichbar ist oder diese sogar übertrifft. Ein weiterer Nutzen des erfindungsgemäßen Mittels ist außerdem die gegenüber Silikonen deutlich bessere biologische Abbaubarkeit der erfindungsgemäßen C8- bis C18-Carbonsäurealkylester.

Im erfindungsgemäßen Haarbehandlungsmittel ist Komponente (A) in einer Menge von vorzugsweise 0,01 bis 10 Gew. %, besonders bevorzugt in einer Menge von 0,05 bis 3,0 Gew.% in einer geeigneten kosmetischen Grundlage enthalten.

Besonders bevorzugt sind Ester der Formel (I), bei denen X und Y gleich oder verschieden sind und entweder eine Einfachbindung, eine Methylengruppe oder eine Hydroxymethylengruppe bedeuten. Insbesondere geeignet sind Citronensäuretriester, Äpfelsäurediester und Weinsäurediester. Beispiele erfindungsgemäß geeigneter C8- bis C18-Alkylester sind die unter der Handelsbezeichnung Cosmacol® ECI (Tri-C12-C13-alkylcitrat), Cosmacol® ETI (Di-C12-C13-Alkyltartrat) und Cosmacol® EMI (Di-C12-C13-alkylmalat) von der Firma Condea Chimica vertriebenen Rohstoffe und der unter der Handelsbezeichnung Biosil® Basics Fluoro Guerbet 3.5 von der Firma Biosil vertriebene Rohstoff Di(octyldodecyl)-fluoroheptyl-citrat.

Das haarfestigende Polymer ist ausgewählt aus synthetischen oder natürlichen, nichtionischen, kationischen, anionischen oder amphoteren Polymeren. Ein derartiges Polymeres, das in Mengen von 0,01 bis 50 Gewichtsprozent, vorzugsweise in Mengen von 0,5 bis 20 Gewichtsprozent im Haarbehandlungsmittel enthalten ist, kann auch aus einem Gemisch mehrerer Polymerer bestehen und durch den Zusatz weiterer Polymerer mit verdickender Wirkung in seinen haarfestigenden Eigenschaften noch modifiziert werden.

Unter haarfestigenden Polymeren werden erfindungsgemäß solche Polymere verstanden, die bei Anwendung in 0,01 bis 5%-iger wässriger, alkoholischer oder wässrigalkoholischer Lösung in der Lage sind, auf dem Haar einen Polymerfilm abzuscheiden und auf diese Weise das Haar zu festigen.

Enthalten die Polymere Säuregruppen, so können diese teilweise oder ganz mit einer geeigneten Base wie z.B. Aminomethylpropanol neutralisiert werden. Enthalten die Polymere basische Gruppen, so können diese teilweise oder ganz mit einer geeigneten Säure wie z.B. Ameisensäure, Pyrrolidoncarbonsäure, Milchsäure usw. neutralisiert werden.

Geeignete synthetische filmbildende, anionische Polymere sind z.B. vernetzte oder unvernetzte Vinylacetat/ Crotonsäure Copolymere, die beispielsweise in Form einer 60%igen Lösung in Isopropanol/Wasser unter der Handelsbezeichnung ARISTOFLEX® von der Firma HOECHST/Deutschland beziehungsweise von der Firma BASF unter dem Handelsnamen LUVISET® CA-66 vertrieben werden. Weitere geeignete anionische Polymere sind zum Beispiel Terpolymere aus Acrylsäure, Alkylacrylat und N-Alkylacrylamid, insbesondere Acrylsäure/Ethylacrylat/N-t-Butylacrylamid Terpolymere oder Terpolymere aus Vinylacetat, Crotonat und Vinylalkanoat, insbesondere Vinylacetat/Crotonat/ Vinylneodecanoat Copolymere.

Kationische Polymere, die in dem erfindungsgemäßen Mittel enthalten sein können,sind z.B. Polyvinylpyrrolidon/Dimethylaminoethylmethacrylat Copolymere; Copolymere aus Polyvinylpyrolidon und Imidazoliminmethochlorid; Terpolymere aus Dimethyldiallylammoniumchlorid, Natriumacrylat und Acrylamid; Terpolymer aus Vinylpyrrolidon, Dimethylaminoethylmethacrylat und Vinylcaprolactam, das von der Firma Amerchol, USA, unter dem Handelsnamen Polymer IR® vertriebene quaternierte Ammoniumsalz der Hydroxyethylcellulose und einem mit Trimethylammonium substituierten Epoxid; Vinylpyrrolidon/Methacrylamidopropyltrimethylammoniumchlorid Copolymer und diquaternäre Polydimethylsiloxane, wie sie von der Firma Goldschmidt, Deutschland, unter dem Handelsnamen Abil® Quat 3272 vertrieben werden.

Geeignete amphotere Polymere sind zum Beispiel Copolymere gebildet aus Alkylacrylamid, insbesondere Octylacrylamid, Alkylaminoalkylmethacrylat, insbesondere t-Butylaminoethylmethacrylate und zwei oder mehr Monomeren bestehend aus Acrylsäure, Methacrylsäure oder deren Ester, wie sie zum Beispiel unter dem Handelsnamen Resyn 28-4910 oder Amphomer LV-71 der Firma NATIONAL STARCH, USA erhältlich sind.

Geeignete synthetische, nichtionische filmbildende, haarfestigende Polymere sind z.B. Homopolymere des Vinylpyrrolidons, sowie Homopolymere des N-Vinylformamids. Weitere geeignete synthetische filmbildende, nicht-ionische, haarfestigende Polymere sind z.B. Copolymerisate aus Vinylpyrrolidon und Vinylacetat, Terpolymere aus Vinylpyrrolidon, Vinylacetat und Vinylpropionat, Polyacrylamide, die beispielsweise unter den Handelsbezeichnungen Akyponine® P 191 von der Firma CHEM-Y, Emmerich, oder Sepigel® 305 von der Firma Seppic vertrieben werden; Polyvinylalkohole, die beispielsweise unter den Handelsbezeichnungen Elvanol® von Du Pont oder Vinol® 523/540 von der Firma Air Products vertrieben werden sowie Polyethylenglykol/ Polypropylenglykol-Copolymere, die beispielsweise unter den Handelsbezeichnungen Ucon® der Union Carbide vertrieben werden.

Geeignete natürliche filmbildende Polymere mit haarfestigender Wirkung sind zum Beispiel Chitosan mit einem Molekulargewicht von 20.000 bis ca. 5 Millionen g/mol. Desweiteren können verschiedene Saccharidtypen verwendet werden wie Polysaccharide oder Gemische aus Oligo-, Mono- und Disacchariden, welche beispielsweise unter dem Handelsnamen C-PUR® von der Firma Cerestar, Brüssel, vertrieben werden. Weitere geeignete, natürliche Polymere sind chinesisches Balsamharz und Cellulosederivate, z.B. Hydroxypropylcellulose mit einem Molekuklargewicht von 30.000 bis 50.000 g/mol, welche beispielsweise unter der Handelsbezeichnung Nisso Sl® von der Firma Lehmann & Voss, Hamburg, vertrieben wird. Ein weiteres natürliches Polymer ist Schellack.

Schellack kann in neutralisierter Form und unneutralisiert zum Einsatz kommen.

Die Konsistenz des erfindungsgemäßen Haarbehandlungsmittels kann beispielsweise durch den Zusatz von Verdickern erhöht werden. Beispiele für Verdicker, die in dem erfindungsgemäßen Mittel enthalten sein können, sind Homopolymere der Acrylsäure mit einem Molekulargewicht von 2.000.000 bis 6.000.000 (Carbopole), z.B. Acrylsäurehomopolymer mit einem Molekulargewicht von 4.000.000 (Carbopol .940). Weitere Verdicker sind beispielsweise die von Goodrich unter dem Handelsnamen Carbopol® ETD 2001 oder von der Firma Protex/Frankreich unter dem Handelsnamen Modarez V 600 PX vertriebene Acrylsäurehomopolymer, das von Hoechst unter dem Handelsnamen Hostacerin® PN 73 vertriebene Polymer aus Acrylsäure und Acrylamid (Natriumsalz mit einem Molekulargewicht von 2.000.000 bis 6.000.000) und Sclerotium Gum. Besonders bevorzugt sind die Copolymeren, der Acrylsäure oder der Methacrylsäure, wie sie z.B. unter dem Handelsnamen Carbopol® 1342 oder Pemulen® TR1 von Goodrich vertrieben werden.

Weitere Beispiele für geeignete Copolymere sind beispielsweise das Acryl- oder Methacrylsäure/Acryloder Methacrylsäurepolyethoxyalkylester Copolymer (INCI-Bezeichnung: Acrylates/ Steareth-20 Methacrylate Copolymer)., wie es von der Firma Rohm und Haas/USA unter den Bezeichungen Acrysol®-22, Acrysol® ICS oder Aculyn®-22 vertrieben wird oder Acryl- oder Methacrylsäure/Polyethoxyalkylallylether Copolymere (INCI-Bezeichnung: Steareth-10 Allyl Ether/Acrylates Copolymer), wie sie von der Firma Allied Colloids, Großbritannien unter der Bezeichnung Salcare® SC 90 vertrieben werden, oder Acryl- oder Methacrylsäure/Itaconsäurepolyethoxyalkylester Copolymere (INCI-Bezeichnungen: Acrylates/Steareth-20 Itaconate Copolymer und Acrylates/Ceteth-20 Itaconate Copolymer), wie sie von der Firma National Starch/USA unter den Bezeichnungen Structure® 2001 und Structure® 3001 vertrieben werden.

Desweiteren können als Verdicker Cellulosen oder Cellulosederivate eingesetzt werden. Beispiele sind Cellulose Gum, ethoxylierte Cellulosen wie das unter der Bezeichnung Natrosol® von der Firma Aqualon oder das unter der Bezeichnung Cellosize® von der Firma Amerchol vertriebene Produkt. Weitere Beispiele für Verdicker sind Stärken oder Gums wie beispielsweise Guar Gum (z.B. N-Hance 3000 Guar von Aqualon), Guar Hydroxypropyltrimoniüm Chloride (z.B. Jaguar C-162 von Rhone-Poulenc oder Cosmedia Guar C 261 von Henkel), Xanthan Gum, wie es z.B. unter der Handelsbezeichnung Keltrol von der Firma Calgon vertrieben wird, Hydroxypropyl Guar (Jaguar HP 120 von Rhone-Poulenc) oder Karaya Gum der Firma TIC Gums.

Als Verdicker können außerdem anorganische Verdicker wie beispielsweise Bentonite oder Hektorite eingesetzt werden oder synthetische kationische Polymere wie z.B. Polyquaternium-31, Polyquaternium-27 oder verdickende kationische Polymere wie sie unter der Handelsbezeichnung Salcare® von der Firma Allied Colloids zu finden sind.

Desweiteren können als Zusätze konditionierende Polymere im erfindungsgemäßen Mittel enthalten sein. Beispiele sind das unter der Handelsbezeichnung Celquat® von der Firma National Starch vertriebene Produkt, Polyquaternium-10, Polyquaternium-24, Steardimonium Hydroxyethyl Cellulose, Methacryloyl Ethyl Betaine/Methacrylates Copolymer, Polymethacrylamidopropyl Trimonium Chlorid, Polyquaternium-2, Polyquaternium-6, Polyquaternium-7, Polyquaternium-18, Polyquaternium-22, Polyquaternium-27, Polyquaternium-39 sowie Polymere mit Siloxaneinheiten wie beispielsweise, Polyquaternium-41, Polyquaternium-42, oder Polyquaternium-80.

Bevorzugte Anwendungen des erfindungsgemäßen Mittels liegen ohne Zusätze von Silikonen vor. Es ist jedoch auch möglich, in silikonhaligen Produkten durch den Einsatz der erfindungsgemäßen Komponente (A) den Anteil an Silikonrohstoffen zu senken. Das erfindungsgemäße Mittel kann wasserlösliche oder wasserunlösliche Silikonverbindungen in einer Konzentration von 0,01 bis 5 Gewichtsprozent enthalten. Besonders bevorzugt sind dabei flüchtige und nichtflüchtige Cyclomethicone und Dimethicone sowie Dimethicon-Copolyole. Beispiele sind: Polydimethylsiloxan (Dimethicon), $\alpha$-Hydro-$\omega$-hydroxypolyoxydimethylsilylen (Dimethiconol), cyclisches Dimethylpolysiloxan (Cyclomethicon), Trimethyl-(octadecyloxy)silan (Stearoxytrimethylsilan), Dimethylsiloxan/Glykol Copolymer (Dimethicon Copolyol), Dimethylsiloxan/Aminoalkylsiloxan Copolymer mit Hydroxyendgruppen (Amodimethicon), Monomethylpolysiloxan mit Laurylseitenketten und Polyoxyethylen- und/oder Polyoxypropylenendketten, (Laurylmethicon Copolyol), Dimethylsiloxan/Glykol Copolymeracetat (Dimethiconcopolyol Acetat) Dimethylsiloxan/Aminoalkylsiloxan Copolymer mit Trimethylsilylendgruppen (Trimethylsilylamodimethicon). Bevorzugte Silikonpolymere sind: Dimethicone, welche beispielsweise von der Firma Wacker, München, unter der Handelsbezeichnung Siloxane F-221 oder von der Firma Dow Corning Europe, Brüssel, unter der Handelsbezeichnung Dow Corning Fluid 200/0,65 cs vertrieben werden; Cyclomethicone, die beispielsweise unter den Handelsbezeichnungen Dow Corning 244 Fluid von der Firma Dow Corning Europe oder Abil® K4 von der Firma Goldschmidt vertrieben werden; Dimethiconole, die beispielsweise unter den Handelsbezeichnungen Silicone Fluid F-212 von der Firma Wacker oder Unisil® SF-R von der Firma UPI vertrieben werden.

Die vorstehend in Klammern angegebenen Bezeichnungen entsprechen der INCI Nomenklatur (International Cosmetic Ingredients), wie sie zur Kennzeichnung kosmetischer Wirk- und Hilfsstoffe bestimmt sind.

Auch Mischungen von Silikonpolymeren sind geeignet, wie z.B. eine Mischung aus Dimethicon und Dimethiconol, die beispielsweise unter der Handelsbezeichnung Dow Corning 1403 Fluid von der Firma Dow Corning Europe vertrieben wird.

Das erfindungsgemäße Mittel kann weitere Konsistenzgeber, wie sie üblicherweise für Cremes eingesetzt werden, enthalten, beispielsweise Fettalkohole und Fettalkoholsulfate wie sie unter der Bezeichnung Lanette® vertrieben werden. Ebenfalls enthalten sein können bei Raumtemperatur flüssige, wachsartige oder feste Polyethylenglykole.

Selbstverständlich kann das erfindungsgemäße Mittel auch weitere übliche kosmetische Zusätze, wie nichtfestigende, nichtionische Polymere, nichtfestigende, anionische Polymere und nichtfestigende, natürliche Polymere sowie deren Kombination in einer Menge von vorzugsweise 0,01 bis 15 Gewichtsprozent; Parfümöle in einer Menge von vorzugsweise 0,01 bis 5 Gewichtsprozent; Trübungsmittel wie z.B. Ethylenglykoldistearat, Styrol/PVP Copolymere oder Polystyrole in einer Menge von vorzugsweise 0,01 bis 5 Gewichtsprozent; Netzmittel, Tenside oder Emulgatoren mit oder ohne Waschaktivität aus den Klassen der anionischen, kationischen, amphoteren oberflächenaktiven Substanzen wie Fettalkoholsulfate, Fettalkoholethersulfate, Fettsäurealkanolamide in einer Menge von vorzugsweise 0,1 bis 20 Gewichtsprozent; ferner Feuchthaltemittel, Farbstoffe, Lichtschutzmittel, Antioxidantien, Glanzgeber und Konservierungsstoffe in einer Menge von vorzugsweise 0,01 bis 10 Gewichtsprozent enthalten.

Das erfindungsgemäße Haarbehandlungsmittel kann als wässrige, alkoholische, wässrig-alkoholische oder als wasserfreie Lösung vorliegen. Geeignete Lösungsmittel sind beispielsweise aliphatische Alkohole mit 1 bis 4 Kohlenstoffatomen oder ein Gemisch von Wasser mit einem der genannten Alkohole. Es können jedoch auch andere organische Lösungsmittel eingesetzt werden, wobei insbesondere unverzweigte oder verzweigte Kohlenwasserstoffe wie Pentan, Hexan, Isopentan und zyklische Kohlenwasserstoffe wie Cyclopentan und Cyclohexan zu nennen sind. Die Lösungsmittel liegen in einer Menge von 0,5 bis 90 Gewichtsprozent, bevorzugt in einer Menge von 5 bis 50 Gewichtsprozent vor.

Das erfindungsgemäße Mittel kann in verschiedenen Applikationsformen Anwendung finden, wie beispielsweise in Aerosolzubereitungen als Schaum oder als Spray, desweiteren als Non-Aerosol, welches mittels einer Pumpe oder als "Pump and Spray" zum Einsatz kommt. Der Einsatz in üblichen O/W und W/O Emulsionen ist ebenso möglich wie in Anwendungsformen als Gel, Wachs oder Mikroemulsion. Wenn das erfindungsgemäße Mittel in Form eines Aerosol-Haarsprays oder Aerosol-Haarlackes vorliegt, so enthält es zusätzlich 15 bis 85 Gewichtsprozent, bevorzugt 25 bis 75 Gewichtsprozent, eines Treibmittels und wird in einem Druckbehälter abgefüllt. Bevorzugte wasserunlösliche Treibmittel sind flüchtige Kohlenwasserstoffe wie beispielsweise Propan und Butan und deren Mischungen sowie fluorierte Treibmittel. Bevorzugtes wasserlösliches Treibmittel ist Dimethylether. Ferner sind bei den in Betracht kommenden Drücken gasförmig vorliegende Treibmittel, wie beispielsweise $N_2$, $N_2O$ und $CO_2$ sowie Gemische der vorstehend genannten Treibmittel geeignet.

Das erfindungsgemäße Mittel zur Festigung der Haare kann auch in Form eines mit Hilfe einer geeigneten mechanisch betriebenen Sprühvorrichtung versprühbaren Non-Aerosol-Haarsprays oder eines Non-Aerosol-Haarlacks vorliegen. Unter mechanischen Sprühvorrichtungen sind solche Vorrichtungen zu verstehen, welche das Versprühen einer Flüssigkeit ohne Verwendung eines Treibmittels ermöglichen. Als geeignete mechanische Sprühvorrichtung kann beispielsweise eine Sprühpumpe oder ein mit einem Sprühventil versehener elastischer Behälter, in dem das erfindungsgemäße kosmetische Mittel unter Druck abgefüllt wird, wobei sich der elastische Behälter ausdehnt und aus dem das Mittel infolge der Kontraktion des elastischen Behälters bei Öffnen des Sprühventils kontinuierlich abgegeben wird, verwendet werden.

Die nachfolgenden Beispiele sollen den Gegenstand der Erfindung näher erläutern.

**Beispiele**

**Beispiel 1:** Schaumfestiger

Muster A und B wurden in ihren Anwendungseigenschaften auf dem Haar durch Halbseitenversuche gegen Muster C und D getestet:

| Muster A | |
|---|---|
| 0,50 g | Di-C12-C13-alkylmalat |
| 2,00 g | Polyvinylpyrrolidon |
| 0,50 g | Chitosan, M = 30.000 bis 70.000 g/mol |
| 0,15 g | Ameisensäure, 85%-ig |
| 0,10 g | Cetyltrimethylammoniumchlorid |
| 0,10 g | Isopropanol |

(fortgesetzt)

| Muster A | |
|---|---|
| 0,30 g | Fettalkoholzuckerether |
| 8,00 g | Propan/Butan 5.0 |
| 88,35 g | Wasser |
| 100,00 g | |

| Muster B | |
|---|---|
| 0,50 g | Di-C12-C13-alkyltartrat |
| 2,00 g | Polyvinylpyrrolidon |
| 0,50 g | Chitosan, M = 30.000 bis 70.000 g/mol |
| 0,15 g | Ameisensäure, 85%-ig |
| 0,10 g | Cetyltrimethylammoniumchlorid |
| 0,10 g | Isopropanol |
| 0,30 g | Fettalkoholzuckerether |
| 8,00 g | Propan/Butan 5.0 |
| 88,35 g | Wasser |
| 100,00 g | |

| Muster C | |
|---|---|
| 0,50 g | Phenyl Trimethicone |
| 2,00 g | Polyvinylpyrrolidon |
| 0,50 g | Chitosan, M = 30.000 bis 70.000 g/mol |
| 0,15 g | Ameisensäure, 85%-ig |
| 0,10 g | Cetyltrimethylammoniumchlorid |
| 0,10 g | Isopropanol |
| 0,30 g | Fettalkoholzuckerether |
| 8,00 g | Propan/Butan 5.0 |
| 88,35 g | Wasser |
| 100,00 g | |

| Muster D | |
|---|---|
| 0,50 g | Dimethicone and Dimethiconol |
| 2,00 g | Polyvinylpyrrolidon |
| 0,50 g | Chitosan, M = 30.000 bis 70.000 g/mol |
| 0,15 g | Ameisensäure, 85%-ig |
| 0,10 g | Cetyltrimethylammoniumchlorid |
| 0,10 g | Isopropanol |
| 0,30 g | Fettalkoholzuckerether |
| 8,00 g | Propan/Butan 5.0 |
| 88,35 g | Wasser |
| 100,00 g | |

Die Muster A und B waren in Bezug auf Glanz und Griffkriterien gleichwertig zu den Mustern C und D. Die Belastung der mit den Mustern A oder B behandelten Haare war geringer, dadurch war auch ein größeres Volumen der Frisur sowie eine verbesserte Festigkeit gegenüber den mit Muster C oder D behandelten Haaren feststellbar. Die Auswaschbarkeit der Muster A und B war hervorragend.

**Beispiel 2:** Nichtwässriger Pumphaarspray ohne Treibgas

Muster A und B wurden in ihren Anwendungseigenschaften auf dem Haar durch Halbseitenversuche gegen Muster C getestet.

| A | B | C | |
|---|---|---|---|
| 94,80 | 94,80 | 94,80 | Ethanol |
| 5,00 | 5,00 | 5,00 | PVP/VA Copolymer |
| 0,20 | 0,00 | 0,00 | Di-C12-C13-alkylmalat |
| 0,00 | 0,20 | 0,00 | Di-C12-C13-alkyltartrat |
| 0,00 | 0,00 | 0, 20 | Phenyl Trimethicone |
| 100,00 | 100,00 | 100,00 | |

Sowohl Muster A als auch Muster B wiesen einen stärkeren Glanz auf, wobei die Frisurerstellung nicht durch eine zusätzliche Belastung des Haares beeinträchtigt wurde. Dies äußerte sich in einer erhöhten Frisurenstabilität durch einen besseren Vernetzungsgrad der Haare von mit Muster A oder Muster B behandeltem Haar im Vergleich zu mit Muster C behandeltem Haar. Die Vernetzung ist ein wichtiges Kriterium für die Stabilität einer Frisur. Die Auswaschbarkeit der Muster A und B aus dem Haar war hervorragend.

**Beispiel 3: Treibgasgetriebener, nichtwässriger Spray**

Muster A und B wurden in ihren Anwendungseigenschaften auf dem Haar durch Halbseitenversuche gegen Muster C getestet.

| A | B | C | |
|---|---|---|---|
| 54,90 | 54,90 | 54,90 | Ethanol |
| 5,00 | 5,00 | 5,00 | Acrylat/Acrylamid Copolymer (Ultrahold® 8), mit AMP neutralisiert |
| 0,10 | 0,00 | 0,00 | Di-C12-C13-alkylmalat |
| 0,00 | 0,10 | 0,00 | Di-C12-C13-alkyltartrat |
| 0,00 | 0,00 | 0,10 | Dimethicone and Dimethiconol |
| 40,00 | 40,00 | 40,00 | Propan/Butan 1,5 bar |
| 100,00 | 100,00 | 100,00 | |

Die mit Muster A und B behandelten Haare wiesen einen stärkeren Glanz auf als die mit Muster C behandelten Haare, ohne dadurch die Frisur stärker zu belasten. Dies äußerte sich in einer stärkeren Vernetzung der mit Muster A oder B erstellten Frisur gegenüber der Frisur, welche mit Muster C erstellt wurde. Die Vernetzung ist ein wichtiges Kriterium für die Stabilität einer Frisur.
Die Auswaschbarkeit von Muster A und B war hervorragend.

**Beispiel 4: Treibgasgetriebener, nichtwässriger Spray**

Muster A und B wurden in ihren Anwendungseigenschaften auf dem Haar durch Halbseitenversuche gegen Muster C getestet.

| A | B | C | |
|---|---|---|---|
| 54,70 | 54,70 | 54,70 | Ethanol |
| 5,00 | 5,00 | 5,00 | Butyl Ester of PVM/MA Copolymer, mit AMP neutralisiert |
| 0,15 | 0,00 | 0,00 | Di-C12-C13-alkylmalat |
| 0,00 | 0,15 | 0,00 | Di-C12-C13-alkyltartrat |
| 0,15 | 0,15 | 0,30 | Dimethicone Copolyol |
| 40,00 | 40,00 | 40,00 | Propan/Butan 1,5 bar |

(fortgesetzt)

| A | B | C | |
|---|---|---|---|
| 100,00 | 100,00 | 100,00 | |

Mit Muster A und B wurde gegenüber Muster C ein verbesserter Glanz sowie eine bessere Vernetzung festgestellt. Die Auswaschbarkeit der Muster A und B ist hervorragend.

**Patentansprüche**

**1.**  Haarbehandlungsmittel mit einem Gehalt an

(A) mindestens einem Ester, welcher mindestens eine alpha-Hydroxycarbonsäuregruppe enthält, die mit einem Alkohol verestert ist, der 8 bis 18 Kohlenstoffatome aufweist und
(B) mindestens einem filmbildenden und haarfestigenden Polymer;

wobei der Ester (A) die allgemeine Formel (I) aufweist

$$\text{HO-C}(XR^1)(YR^2)\text{-CO}_2R^3 \qquad \text{(I)}$$

wobei $R^1$ die Gruppe $CO_2R^4$ bedeutet, $R^2$ ausgewählt ist aus H und $CO_2R^4$, $R^3$ und $R^4$ gleich oder verschieden sein können und ausgewählt sind aus verzweigten oder unverzweigten Alkylgruppen mit 8 bis 18 Kohlenstoffatomen, wobei die Alkylgruppen weitere Substituenten wie Hydroxygruppen, Aminogruppen oder Halogene tragen können und X und Y gleich oder verschieden sein können und entweder eine Einfachbindung darstellen oder ausgewählt sind aus verzweigten oder unverzweigten Alkylengruppen, wobei die Alkylengruppen weitere Substituenten wie Hydroxygruppen, Aminogruppen oder Halogene tragen können;
und/oder wobei das haarfestigende Polymer (B) nichtionisch, kationisch, anionisch oder amphoter sein kann, wobei nichtionische synthetische Polymere ausgewählt sind aus Homopolymeren des N-Vinylformamids, Copolymeren aus Vinylpyrrolidon und Vinylacetat, Terpolymeren aus Vinylpyrrolidon, Vinylacetat und Vinylpropionat, Polyacrylamiden, Polyvinylalkoholen und Polyethylenglykol/Polypropylenglykol Copolymeren;
ausgenommen einem Haarbehandlungsmittel mit einem Gehalt an 0,3 Gew.% einer Mischung aus Isostearylcitrat, Isostearylglycolat, Isostearyllactat und Isostearylmalat in Kombination mit entweder 2 Gew.% Polyquaternium-37, 0,5 Gew.% Polysilicone-8 oder 0,75 Gew.% Hydroxyethylcellulose.

**2.**  Mittel nach Anspruch 1, **dadurch gekennzeichnet, daß** X und Y gleich oder verschieden sein können und entweder eine Einfachbindung, eine Methylengruppe oder eine Hydroxymethylengruppe bedeuten.

**3.**  Mittel nach einem der vorstehenden Ansprüche, **dadurch gekennzeichnet, daß** Komponente (A) ausgewählt ist aus Citronensäuretriestern, Äpfelsäurediestern und Weinsäurediestern.

**4.**  Mittel nach einem der vorstehenden Ansprüche, **dadurch gekennzeichnet, daß** Komponente (A) in einer Menge von 0,01 bis 10 Gewichtsprozent vorliegt.

**5.**  Mittel nach einem der vorstehenden Ansprüche, **dadurch gekennzeichnet, daß** Komponente (B) in einer Menge von 0,01 bis 50 Gewichtsprozent vorliegt.

**6.**  Verwendung von Estern, welche mindestens eine alpha-Hydroxycarbonsäuregruppe enthalten, die mit einem Alkohol verestert ist, der 8 bis 18 Kohlenstoffatome aufweist und wobei die Ester die allgemeine Formel (I) aufweisen

$$\text{HO-C}(XR^1)(YR^2)\text{-CO}_2R^3 \qquad \text{(I)}$$

wobei $R^1$ die Gruppe $CO_2R^4$ bedeutet, $R^2$ ausgewählt ist aus H und $CO_2R^4$, $R^3$ und $R^4$ gleich oder verschieden sein können und ausgewählt sind aus verzweigten oder unverzweigten Alkylgruppen mit 8 bis 18 Kohlenstoffatomen, wobei die Alkylgruppen weitere Substituenten wie Hydroxygruppen, Aminogruppen oder Halogene tragen können und X und Y gleich oder verschieden sein können und entweder eine Einfachbindung darstellen oder ausgewählt sind aus verzweigten oder unverzweigten Alkylengruppen, wobei die Alkylengruppen weitere Substituenten wie Hydroxygruppen, Aminogruppen oder Halogene tragen können, in Haarstylingmitteln.

**Claims**

1. Hair treatment agent with a content of

    (A) at least one ester which comprises at least one alpha-hydroxycarboxylic acid group which is esterified with an alcohol which has 8 to 18 carbon atoms and
    (B) at least one film-forming and hair-setting polymer;

    where the ester (A) has the general formula (I)

$$HO-C(XR^1)(YR^2)-CO_2R^3 \qquad (I)$$

    where $R^1$ is the group $CO_2R^4$, $R^2$ is chosen from H and $CO_2R^4$, $R^3$ and $R^4$ may be identical or different and are chosen from branched or unbranched alkyl groups having 8 to 18 carbon atoms, where the alkyl groups may carry further substituents, such as hydroxy groups, amino groups or halogens, and X and Y may be identical or different and either represent a single bond or are chosen from branched or unbranched alkylene groups, where the alkylene groups may carry further substituents, such as hydroxy groups, amino groups or halogens;
    and/or where the hair-setting polymer (B) may be nonionic, cationic, anionic or amphoteric, where nonionic synthetic polymers are chosen from homopolymers of N-vinylformamide, copolymers of vinylpyrrolidone and vinyl acetate, terpolymers of vinylpyrrolidone, vinyl acetate and vinyl propionate, polyacrylamides, polyvinyl alcohols and polyethylene glycol/polypropylene glycol copolymers;
    with the exception of a hair treatment agent with a content of 0.3% by weight of a mixture of isostearyl citrate, isostearyl glycolate, isostearyl lactate and isostearyl malate in combination with either 2% by weight of polyquaternium-37, 0.5% by weight of polysilicone-8 or 0.75% by weight of hydroxyethylcellulose.

2. Agent according to Claim 1, **characterized in that** X and Y may be identical or different and are either a single bond, a methylene group or a hydroxymethylene group.

3. Agent according to one of the preceding claims, **characterized in that** component (A) is chosen from citric triesters, malic diesters and tartaric diesters.

4. Agent according to one of the preceding claims, **characterized in that** component (A) is present in an amount of from 0.01 to 10% by weight.

5. Agent according to one of the preceding claims, **characterized in that** component (B) is present in an amount of from 0.01 to 50% by weight.

6. The use of esters which contain at least one alpha-hydroxycarboxylic acid group which is esterified with an alcohol which has 8 to 18 carbon atoms and where the esters have the general formula (I)

$$HO-C(XR^1)(YR^2)-CO_2R^3 \qquad (I)$$

    where $R^1$ is the group $CO_2R^4$, $R^2$ is chosen from H and $CO_2R^4$, $R^3$ and $R^4$ may be identical or different and are chosen from branched or unbranched alkyl groups having 8 to 18 carbon atoms, where the alkyl groups may carry further substituents, such as hydroxy groups, amino groups or halogens, and X and Y may be identical or different and either represent a single bond or are chosen from branched or unbranched alkylene groups, where the alkylene groups may carry further substituents, such as hydroxy groups, amino groups or halogens, in hairstyling products.

**Revendications**

1. Composition de traitement capillaire ayant une teneur en

(A) au moins un ester qui comporte au moins un groupe α-hydroxycarboxy qui est estérifié par un alcool qui a de 8 à 18 atomes de carbone et
(B) au moins un polymère filmogène et fixant les cheveux ;

l'ester (A) correspondant à la formule générale (I)

$$HO\text{-}C(XR^1)(YR^2)\text{-}CO_2R^3 \tag{I}$$

dans laquelle $R^1$ représente le groupe $CO_2R^4$, $R^2$ est choisi parmi H et $CO_2R^4$, $R^3$ et $R^4$ peuvent être identiques ou différents et sont choisis parmi des groupes alkyle ramifiés ou non ramifiés ayant de 8 à 18 atomes de carbone, les groupes alkyle pouvant porter d'autres substituants tels que des groupes hydroxy, des groupes amino ou des atomes d'halogène et X et Y pouvant être identiques ou différents et soit représentant une liaison simple, soit étant choisis parmi des groupes alkylène ramifiés ou non ramifiés, les groupes alkylène pouvant porter d'autres substituants tels que des groupes hydroxy, des groupes amino ou des atomes d'halogène ;
et/ou le polymère (B) fixant les cheveux pouvant être non ionique, cationique, anionique ou amphotère, les polymères synthétiques non ioniques étant choisis parmi les homopolymères du N-vinylformamide, les copolymères de vinylpyrrolidone et acétate de vinyle, les terpolymères de vinylpyrrolidone, acétate de vinyle et propionate de vinyle, les polyacrylamides, les poly(alcool vinylique)s et les copolymères polyéthylèneglycol/polypropylèneglycol ; à l'exception d'une composition de traitement capillaire ayant une teneur de 0,3 % en poids en un mélange de citrate d'isostéaryle, glycolate d'isostéaryle, lactate d'isostéaryle et malate d'isostéaryle en association avec soit 2 % en poids de polyquaternium-37, soit 0,5 % en poids de polysilicone-8 ou 0,75 % en poids d'hydroxyéthylcellulose.

2. Composition selon la revendication 1, **caractérisée en ce que** X et Y peuvent être identiques ou différents et représentent soit une liaison simple, soit un groupe méthylène ou un groupe hydroxyméthylène.

3. Composition selon l'une quelconque des revendications précédentes, **caractérisée en ce que** le composant (A) est choisi parmi les triesters d'acide citrique, les diesters d'acide malique et les diesters d'acide tartrique.

4. Composition selon l'une quelconque des revendications précédentes, **caractérisée en ce que** le composant (A) est présent en une quantité de 0,01 à 10 % en poids.

5. Composition selon l'une quelconque des revendications précédentes, **caractérisée en ce que** le composant (B) est présent en une quantité de 0,01 à 50 % en poids.

6. Utilisation d'esters qui comportent au moins un groupe α-hydroxycarboxy qui est estérifié par un alcool qui a de 8 à 18 atomes de carbone et les esters correspondant à la formule générale (I)

$$HO\text{-}C(XR^1)(YR^2)\text{-}CO_2R^3 \tag{I}$$

dans laquelle $R^1$ représente le groupe $CO_2R^4$, $R^2$ est choisi parmi H et $CO_2R^4$, $R^3$ et $R^4$ peuvent être identiques ou différents et sont choisis parmi des groupes alkyle ramifiés ou non ramifiés ayant de 8 à 18 atomes de carbone, les groupes alkyle pouvant porter d'autres substituants tels que des groupes hydroxy, des groupes amino ou des atomes d'halogène et X et Y pouvant être identiques ou différents et soit représentant une liaison simple, soit étant choisis parmi des groupes alkylène ramifiés ou non ramifiés, les groupes alkylène pouvant porter d'autres substituants tels que des groupes hydroxy, des groupes amino ou des atomes d'halogène, dans des compositions de coiffage.